# EUROPÄISCHE PATENTSCHRIFT

(11) **EP 0 387 604 B1**
(45) Veröffentlichungstag und Bekanntmachung des Hinweises auf die Patenterteilung: **30.06.1993**
(21) Anmeldenummer: 90103843.0
(22) Anmeldetag: 28.02.1990
(51) Int. Cl.: B01D 53/28, B01J 20/32

(54) **Trockenmittel-Tabletten**
Desiccant tablets
Gélules déshydratantes

(30) Priorität: 11.03.1989 DE 3907973
(43) Veröffentlichungstag der Anmeldung: 19.09.1990
(73) Patentinhaber: MERCK PATENT GmbH, D-64271 Darmstadt (DE)
(72) Erfinder: Henzler, Peter, Dr., D-6056 Heusenstamm (DE); Loebich, Friedrich, Dr., D-6100 Darmstadt (DE); Oelrich, Eckhard, Dr., DD-6101 Rossdorf (DE); Sassor, Jürgen, D-6100 Darmstadt (DE); Schleehahn, Michael, D-6100 Darmstadt 13 (DE)

(56) Entgegenhaltungen:
- US-A- 4 224 366
- CHEMICAL ABSTRACTS, Band 109, 1988, Seite 701, Zusammenfassung Nr. 229138t, Columbus, Ohio, US;
- PATENT ABSTRACTS OF JAPAN, Band 5, Nr. 62 (C-52)[734], 25. April 1981;
- PATENT ABSTRACTS OF JAPAN, Band 8, Nr. 199 (C-242)[1636], 12. September 1984;
- PATENT ABSTRACTS OF JAPAN, Band 9, Nr. 160 (C-289)[1883], 4. Juli 1985, Seite 117 C 289;

## Beschreibung

Die Erfindung betrifft eine Trockenmittel-Tablette (TT) mit einer Umhüllung, wobei diese Umhüllung als wesentlichen Bestandteil einen wasserlöslichen Filmbildner enthält.

Es ist bekannt, TT bei der Verpackung feuchtigkeitsempfindlicher Arzneimittel einzusetzen. So können z.B. feuchtigkeitsempfindliche Tabletten in wasserdampfundurchlässige Verpackungen eingearbeitet werden, wobei die Verpackung neben den Arzneimittel-Tabletten zusätzlich eine Trockenmitteleinheit in Form einer Tablette enthält.

Bekannte TT haben den Nachteil, daß sie, z.B. durch Erschütterungen beim Transport, einen pulverförmigen Abrieb erzeugen, der das Arzneimittel verunreinigen kann. Außerdem besteht eine gewisse Verwechslungsgefahr zwischen den TT und den Arzneimittel-Tabletten.

In der JP-61291021 sind TT beschrieben, die mit flüssigem Harz oder Gummi, z.B. auch Silicongummi, überzogen werden und einen geringeren pulverförmigen Abrieb aufweisen. Diese Tabletten zeigen wieder andere Nachteile, insbesondere bei ihrer Herstellung, die in nichtwässerigem Medium erfolgen muß.

Der Erfindung lag die Aufgabe zugrunde, eine TT bereitzustellen, die die Nachteile der bekannten TT nicht oder nur in geringerem Maße aufweist. Ein wesentlicher Teil der Aufgabe war es, ein Umhüllungsverfahren aufzufinden, bei welchem kein organisches Lösungsmittel eingesetzt wird.

Es wurde gefunden, daß man die TT mit einer Umhüllung versehen kann, die als wesentlichen Bestandteil einen wasserlöslichen Filmbildner enthält. Diese Umhüllung verhindert den Abrieb bei der Handhabung, wobei Trocknungsgeschwindigkeit und -kapazität nicht wesentlich vermindert werden.

Gegenstand der Erfindung ist eine TT mit Umhüllung, dadurch gekennzeichnet, daß die Umhüllung als wesentlichen Bestandteil einen wasserlöslichen Filmbildner enthält.

Als Trockenmittel eignen sich insbesondere solche, die bei der Wasseraufnahme ihr Volumen nicht ändern (nicht quellen), vor allem Kieselgele, in erster Linie engporige Kieselgele mit einem Korngrößenbereich zwischen etwa 200 und 400 µm. Auch Molekularsiebe, Aluminiumoxide oder Trockentone sind geeignet.

Das Trockenmittel kann in üblicher Weise zu Tabletten von etwa 50-5000 mg, vorzugsweise 100-500 mg, gepreßt werden. Die Tablettenkerne können übliche Bindemittel, z.B. Polyvinylpyrrolidon (PVP), und sonstige übliche Zusatzstoffe enthalten. Verwendet man PVP, so ist ein Gehalt von 3-10 Gew.% bevorzugt (bezogen auf das Gesamtgewicht der Tablettenkerne). Bei höheren PVP-Anteilen sind die Tabletten härter, bei niedrigeren PVP-Anteilen sind sie weicher.

Unter den wasserlöslichen Filmbildnern sind Cellulosederivate bevorzugt. Insbesondere ist Methylhydroxypropylcellulose gut geeignet, ferner z.B. Methylcellulose, Hydroxypropylcellulose, Natriumcarboxymethylcellulose. Es ist auch möglich, daß die Umhüllung mehrere verschiedene wasserlösliche Cellulosederivate enthält. Andere geeignete Filmbildner sind Polyvinylalkohol und PVP.

Die Umhüllung der TT kann erfindungsgemäß zusätzlich einen oder mehrere Weichmacher enthalten. Unter diesen sind ebenfalls die wasserlöslichen bevorzugt, insbesondere Polyalkylenglykole, zweckmäßig Polyethylenglykole wie Polyethylenglykol 400 oder Polyethylenglykol 6000. Ferner seien genannt Alkylenglykole wie Propylenglykol (1,2-Propandiol), Ester, z.B. Trialkylcitrate wie Triethylcitrat, Polyoxyethylen-Polyoxypropylen-Blockpolymere.

Die Umhüllung der TT kann erfindungsgemäß zusätzlich ein oder mehrere Pigmente enthalten. Mit Hilfe dieser Pigmente können die TT eingefärbt werden, zweckmäßig so, daß sie gegebenenfalls von den in der gleichen Packung befindlichen Arzneimittel-Tabletten deutlich unterschieden werden können. Als Pigmente eignen sich alle in der Galenik üblichen, z.B. Oxide wie Titandioxid oder Eisenoxid; Carbonate wie Calciumcarbonat; Farblacke wie Chinolingelblack E 104, Amaranthlack E 123 oder Indigotinlack E 132.

Diese Pigmente beeinflussen die Wasserdampfdurchlässigkeit der Hülle praktisch nicht.

Die Umhüllung kann weitere Zusatzstoffe enthalten, z.B. Gleitmittel wie Talk oder Silikonöl, das letzte auch in Form einer Emulsion.

Die Umhüllung wird zweckmäßig in einer Stärke von etwa 0,7 bis 2,5 mg/cm² aufgetragen. Ihr Anteil, bezogen auf das Gesamtgewicht der TT, beträgt etwa 1-5 Gew.%. Dabei besitzen die TT zweckmäßig Gesamtgewichte zwischen etwa 100 und 1000 mg.

Die Umhüllung wird erfindungsgemäß in üblicher Weise aufgetragen. Zweckmäßig verwendet man wässerige Lösungen oder Suspensionen, die die gewünschten Bestandteile enthalten und auf die Tablettenkerne aufgesprüht werden, vorzugsweise bei Temperaturen zwischen 20 und 100°, insbesondere zwischen 50 und 80°. Infolge der Wasseraufnahmefähigkeit der TT kann die Aufbringung der Filmhülle in etwa 1/3 der üblicherweise benötigten Zeit erfolgen. Nach der Umhüllung der TT kann das enthaltene Wasser ohne wesentliche Beeinträchtigung durch die Filmhülle entfernt werden.

Eine Trocknung erfolgt zweckmäßig bei Temperaturen zwischen 20 und 120°, insbesondere zwischen 50 und 80°, unter vermindertem Druck.

### Beispiel

Man löst 2,0 kg Methylhydroxypropylcellulose und 0,467 kg Polyethylenglykol 400 in 22,0 kg Wasser. Zu dieser Lösung gibt man eine Suspension von 0,333 kg Titan(IV)-oxid und 0,733 kg Eisenoxidrot in 7,8 kg Wasser, mischt und siebt (Sieb 180, Ph.Eur.).

Die so erhaltene Suspension wird unter Rühren auf 90 kg auf 70° vorgewärmte Tablettenkerne aufgesprüht, wobei jeder einzelne Tablettenkern aus 190 mg Kieselgel, Typ E 0-400, und 10 mg PVP besteht.

Die erhaltenen überzogenen TT werden 24 Std. bei 70-80° unter vermindertem Druck getrocknet.

Ein Abrieb (Friabilator, Typ "Roche", 10 Min.) ist nicht nachweisbar.

## Patentansprüche

1. Trockenmittel-Tablette mit Umhüllung, dadurch gekennzeichnet, daß die Umhüllung als wesentlichen Bestandteil einen wasserlöslichen Filmbildner enthält.

2. Trockenmittel-Tablette nach Anspruch 1, dadurch gekennzeichnet, daß die Umhüllung zusätzlich mindestens einen Weichmacher enthält.

3. Trockenmittel-Tablette nach mindestens einem der Ansprüche 1 oder 2, dadurch gekennzeichnet, daß die Umhüllung zusätzlich mindestens ein Pigment enthält.

4. Verfahren zur Herstellung einer Trockenmittel-Tablette mit Umhüllung, dadurch gekennzeichnet, daß man eine Trockenmittel-Tablette mit einer Umhüllung versieht, die als wesentlichen Bestandteil einen wasserlöslichen Filmbildner enthält.

5. Verwendung einer Trockenmittel-Tablette mit einer als wesentlichen Bestandteil einen wasserlöslichen Filmbildner enthaltenden Umhüllung bei der Verpackung von Arzneimitteln.

## Claims

1. Coated desiccant tablet, characterized in that the coating contains as the essential constituent a water-soluble film former.

2. Desiccant tablet according to Claim 1, characterized in that the coating additionally contains at least one plasticizer.

3. Desiccant tablet according to at least one of Claims 1 or 2, characterized in that the coating additionally contains at least one pigment.

4. Process for the preparation of a coated desiccant tablet, characterized in that a desiccant tablet is provided with a coating which as the essential constituent contains a water-soluble film former.

5. Use of a desiccant tablet coated with a coating containing as the essential constituent a water-soluble film former for the packaging of pharmaceuticals.

## Revendications

1. Tablette déshydratante avec enrobage, caractérisée en ce que l'enrobage contient comme constituant essentiel un agent filmogène soluble dans l'eau.

2. Tablette déshydratante selon la revendication 1, caractérisée en ce que l'enrobage contient en outre au moins un plastifiant.

3. Tablette déshydratante selon au moins l'une des revendications 1 à 2, caractérisée en ce que l'enrobage contient en outre au moins un pigment.

4. Procédé de fabrication de tablettes déshydratantes avec enrobage, caractérisé en ce que l'on recouvre une tablette déshydratante d'un enrobage contenant comme constituant essentiel un agent filmogène soluble dans l'eau.

5. Utilisation d'une tablette déshydratante avec un enrobage contenant comme constituant essentiel un agent filmogène soluble dans l'eau pour l'emballage des médicaments.
